# EUROPEAN PATENT SPECIFICATION

(11) **EP 1 262 154 B1**
(45) Date of publication and mention of the grant of the patent: **09.02.2005**
(21) Application number: 02011875.8
(22) Date of filing: 28.05.2002
(51) Int. Cl.: A61F 2/16

(54) **Intraocular lens injection instrument**
Instrument zum Einführen einer Intraokularlinse
Instrument d'implantation d'une lentille intraoculaire

(30) Priority: 01.06.2001 JP 2001167326
(43) Date of publication of application: 04.12.2002
(73) Proprietor: Nidek Co., Ltd., Gamagori-shi, Aichi 443-0035 (JP)
(72) Inventor: Sunada, Tustomu, Toyohashi-shi, Aichi, 441-3101 (JP)
(74) Representative: Prüfer, Lutz H., Dipl.-Phys.

(56) References cited:
- WO-A-00/62712
- US-A- 5 474 562
- US-A- 5 702 402
- US-A- 5 928 245

## Description

### 1. Field of the Invention

The present invention relates to an instrument for injecting an intraocular lens into an eye of a patient.

### 2. Description of Related Art

As one of operative treatments for cataract, generally used is a method of removing a crystalline lens from an eye of a patient and then injecting an intraocular lens in place of the crystalline lens. To inject the intraocular lens, the following steps are taken: first making an incision in an eyeball of a patient' s eye; fragmenting and aspirating a clouded crystalline lens through the incision by for example an ultrasonic cataract-surgery device (a phaco-emulsification device); and then injecting the intraocular lens into the eye through the incision to implant it in place of the crystalline lens.

If a large incision is made, it may become a burden on the eyeball and also cause astigmatism of the patient's eye after the operation. To prevent such the disadvantages, an intraocular lens injecting instrument called an injector is used to inject a foldable intraocular lens into an eye through a smaller incision. In this injector, the foldable intraocular lens held in a housing of the injector is pushed toward the tip of the injector while being folded into a smaller shape. Thereafter, the intraocular lens is pushed out of the tip of the injector inserted in the eye through the incision and is spread (unfolded) in the eye. The tip of such the injector is desired to be smallest (finest) in shape and size in order to form as small an incision as possible.

The conventional injector, however, is merely configured so that a tube 100 through which an intraocular lens 40 is to be pushed has a diameter (inside diameter) gradually becoming smaller (finer) toward a tip 100a as shown in Fig. 5A.

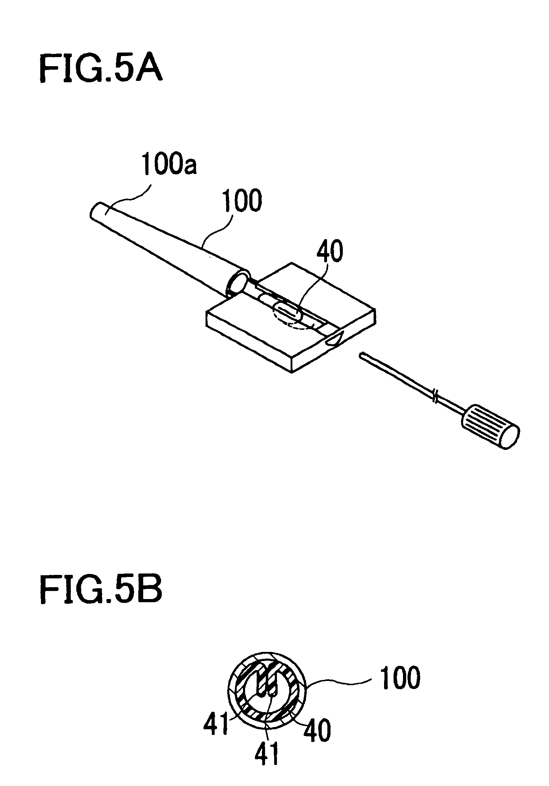

Thus, the intraocular lens 40, which is pushed through the tube 100, would be folded so that both edges 41 of an optical part thereof in diametrical section orthogonal to the direction in which the lens 40 is pushed are bent inside as shown in Fig. 5B. If folded in such a way, the intraocular lens 40 could not be brought into a sufficiently small (compact) state.

From US 5,928,245 an intraocular lens injecting instrument for injecting a foldable intraocular lens into an eye according to the preamble of claim 1 can be taken. A protrusion is use to fold a center portion of an intraocular lens in a lens delivery passage way. Opposite edges of the lens fold upwardly into opposite groove when the lens is moved along the insertion tube.

From WO 00/62712 A1 an asymmetric intraocular lens injecting cartridge can be taken. A bore in the injection cartridge is ramped on one side and contains a flat ledge or a shelf on the other side at the entrance. The bore is asymmetric to roll or fold the edges of a lens to some degree. The bore is symmetric in the insertion tube towards the end.

### SUMMARY OF THE INVENTION

The present invention has been made in view of the above circumstances and has as an object to overcome the above problems and to provide an interocular lens injecting instrument capable of bringing an intraocular lens into a smaller state during the injection into a patient's eye as compared with the conventional instruments.

This object is solved by an intraocular lens injecting instrument according to claim 1.

Further developments of the present invention are given in the dependent claims.

### BRIEF DESCRIPTION OF THE DRAWINGS

The accompanying drawings, which are incorporated in and constitute a part of this specification illustrate an embodiment of the invention and, together with the description, serve to explain the objects, advantages and principles of the invention.

In the drawings,
Fig. 1A is a schematic perspective view of an intraocular lens injection instrument according to an embodiment of the present invention;
Fig. 1B is a plane view of the instrument of Fig. 1A;
Fig. 2 is a sectional view of an insertion tube seen from a setting part side;
Fig. 3 is a sectional view of a main body taken along the line A-A in the Fig. 1B;
Figs. 4A to 4C are sectional views each showing in sequence how an intraocular lens is rolled in the tube;
Fig. 5A is a perspective schematic view of an intraocular lens injection instrument in a prior art; and
Fig. 5B is a sectional view of a tube of the instrument of Fig. 5A.

### DETAILED DESCRIPTION OF THE PREFERRED EMBODIMENTS

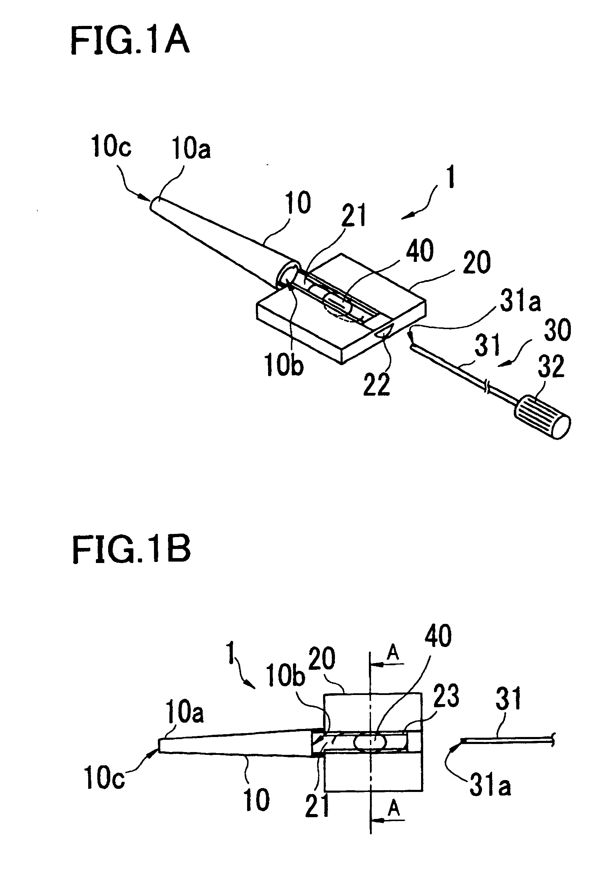

A detailed description of a preferred embodiment of an intraocular lens injection instrument embodying the present invention will now be given referring to the accompanying drawings. Fig. 1A is a schematic perspective view of the instrument in the present embodiment and Fig. 1B is a plane view of the instrument of Fig. 1A.

Numeral 1 is a main body of the injection instrument. This main body 1 includes, in order from the side to be insertable in an eye of a patient, an insertion tube 10 which may be inserted in an incision made in the eye, a setting part 20 joined to the tube 10 and used to set an intraocular lens 40 in the main body 1, and a rodlike pushing member 30 for pushing the intraocular lens 40 set in the setting part 20 to the outside through the tube 10. It is to be noted that the tube 10 has an inlet 10b and an outlet 10c.

The tube 10 is of a whole outer shape of substantially a truncated cone. The tube 10 also has a hollow tubular, tapered shape with its inside and outside diameters gradually becoming smaller or finer toward the tip 10a, that is, from the inlet 10b toward the outlet 10c. In other words, an opening diameter of the tip 10a (the outlet 10c) of the tube 10 is designed to be smaller than that of the other end (the inlet 10b) of the tube 10 joined to the setting part 20.

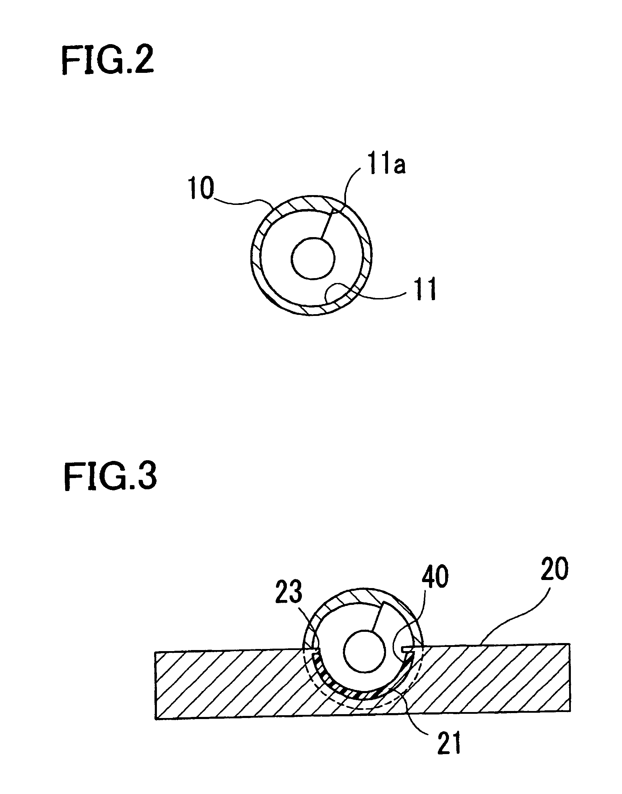

Fig. 2 is a cross-sectional view of the tube 10 seen from the setting part 20 (or the inlet 10b) side, perpendicular to the center axis of the tube 10. Numeral 11 is an inner wall of the tube 10. This inner wall 11 serves to guide the intraocular lens 40 up to the tip 10a of the tube 10, from the inlet 10b to the outlet 10c, and simultaneously to lead a direction to roll the intraocular lens 40. Numeral 11a is a shoulder provided in the inner wall 11. This shoulder 11a is formed in the inner wall 11, providing a stepped portion in a cross-section perpendicular to the center axis of the tube 10, and formed continuously in the inner wall 11 in the axial direction, or a tapering direction, of the tube 10 over its entire length. An edge (peripheral edge) of an optical part of the intraocular lens 40, which is an end in diametrical section orthogonal to the direction in which the lens 40 is guided to the tip 10a of the tube 10, is caught on the shoulder 11a, whereby to restrict a rolling motion of the intraocular lens 40 in one direction along the inner wall 11 in the cross-section but allow only the rolling motion of the lens 40 in the other direction (an opposite direction). The inner wall 11 except for the straight portion of the shoulder 11a has a curved shape in the cross-section, which makes it easy to move the edge of the intraocular lens 40 whose rolling motion is enabled on the inward side of the edge whose rolling motion is disabled by the shoulder 11a.

The shoulder 11a is formed with a height equal to or larger than the thickness of the edge of the optical part of the intraocular lens 40 so that the edge is securely caught on the shoulder 11a. The shoulder 11a is required only to be formed in a position where one edge of the intraocular lens 40 can butt against the shoulder 11a before the both edges butt against each other during the rolling motion of the intraocular lens 40. This rolling motion of the intraocular lens 40 will be described in detail later.

Next, the structures of the setting part 20 and the pushing member 30 will be explained.

The setting part 20 entirely has a box-like shape in which a guide slot 21 is provided opening at both end faces of the setting part 20 in its longitudinal direction. This guide slot 21 is used to delivery the intraocular lens 40 inserted therein to the tube 10. One end of the guide slot 21 is joined to the tube 10 (the inlet 10b) and the other end forms an opening 22 on a short side surface of the setting part 20. The intraocular lens 40 is inserted in the guide slot 21 from the opening 22.

Fig. 3 is a cross-sectional view of the main body 1 taken along the line A-A in Fig. 1B.

As shown in Fig. 3, the guide slot 21 is designed to have a width slightly smaller than the diameter of the optical part of the intraocular lens 40 and a curved bottom surface having a nearly U-shaped cross-section. The thus shaped guide slot 21 defines a direction in which the intraocular lens 40 is rolled after delivered (inserted) into the tube 10. The shoulder 11a is provided above (in Fig. 3) the guide slot 21. Numeral 23 is a protective portion formed on both upper edges of the guide slot 21 to prevent the intraocular lens 40 set in the guide slot 21 from unexpectedly popping out.

The pushing member 30 is constructed of a thin rod 31 for pushing the intraocular lens 40 placed in the guide slot 21 to the outside from the tip 10a (the outlet 10c) of the tube 10 and a grip 32 thicker than the thin rod 31 which makes the pushing member 30 easy to hold. An end 31a of the thin rod 31 is made of relatively soft resin material in order to prevent damage to the intraocular lens 40. The thin rod 31 is of a length enough to allow the end 31a to protrude from the tip 10a (the outlet 10c) of the tube 10 when the rod 31 is inserted through the setting part 20.

The operation of the intraocular lens injection instrument constructed as above will be mentioned below.

At first, a proper amount of viscoelastic material (for instance, hyaluronate sodium) is applied in advance to the intraocular lens 40 and some portions of the main body 1 which the intraocular lens 40 will contact during passage. Then, an operator (surgeon) pinches the intraocular lens 40 by the use of tweezers or the like and inserts it in the guide slot 21 from the opening 22. At this time, the intraocular lens 40 has to be set along the shape of the guide slot 21, that is, to be slightly deformed into a nearly U-shape in section.

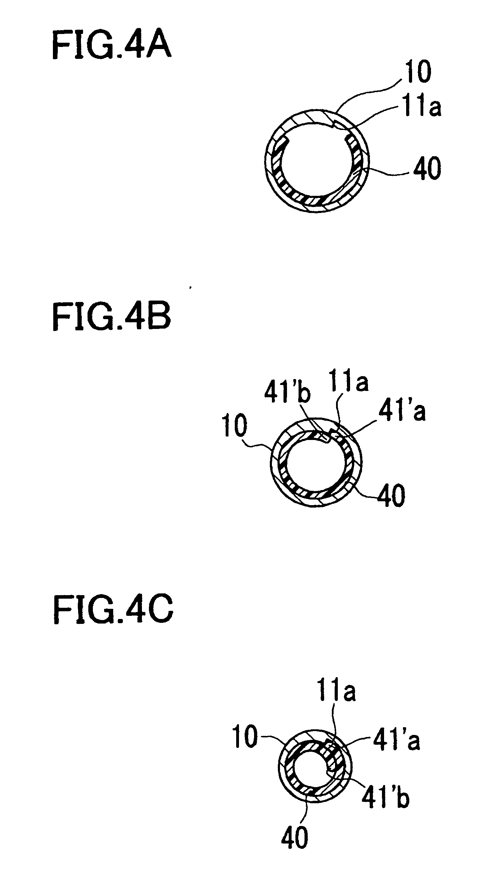

The operator then inserts the end 31a of the pushing member 30 from the opening 22 and pushes the intraocular lens 40 into the tube 10. When the intraocular lens 40 is moved into the tube 10 with its diameter becoming smaller, the intraocular lens 40 is rolled along the inner wall 11 of the tube 10 as shown in Fig. 4A. The lens 40 is previously set in the nearly U-shape in section in the guide slot 21 as above, so that the lens 40 can become rolled smoothly in a U-shape, without being deformed in an inverted U-shape, even when the lens 40 is passed through the tapered tube 10 with its inner diameter becoming gradually smaller.

When the intraocular lens 40 is further pushed by the pushing member 30, where the diameter of the tube 10 becomes further smaller, an edge 41'a of the intraocular lens 40 being rolled along the inner wall 11 butts against the shoulder 11a as shown in Fig. 4B. The edge 41'a is stopped from being further rolled by the shoulder 11a and thus locked in this position.

When the intraocular lens 40 is pushed to move through the tube 10 with its diameter becoming still further smaller, only the other edge 41'b of the intraocular lens 40 is rolled. As shown in Fig. 4C, accordingly, the intraocular lens 40 is further rolled in one direction (into a spiral shape in section) so that the edge 41'b curves on the inward side of the edge 41'a.

As above, the intraocular lens 40 can be rolled in one direction, which can make the size of the rolled state smaller as compared with the conventional folded state shown in Fig. 5B. Accordingly, the tip 10a can be designed to be smaller (finer) in diameter than the conventional one.

The present invention may be embodied in other specific forms without departing from the essential characteristics thereof.

For instance, the tip 10a may be formed with a cutout to make it easy to push the intraocular lens 40 out of the tip 10a.

In the above embodiment, the shoulder 11a of the inner wall 11 is provided over the tube 10 in the axial direction, or the tapering direction. Alternatively, the shoulder 11a may be formed with only a length enough to make the edge 41'b of the intraocular lens 40 move on the inward side of the other edge 41'a during the rolling motion of the lens 40.

As explained above, the intraocular lens injection instrument according to the present invention can bring the intraocular lens into a smaller state during injection into the eye as compared with the conventional one. This allows a design of a finer tip of the intraocular lens injection instrument than that of the conventional instrument. As a result, it is sufficient to make a small incision in the patient's eye.

While the presently preferred embodiment of the present invention has been shown and described, it is to be understood that this disclosure is for the purpose of illustration and that various changes and modifications may be made without departing from the scope of the invention as set forth in the appended claims.

## Claims

1. An intraocular lens injection instrument for injecting a foldable intraocular lens (40) into an eye, including:
an insertion tube (10) which is insertable through an incision made in an eyeball, the tube (10) including an inlet (10b), an outlet (10c), and a tapered inner wall portion (11) having an inside diameter that becomes gradually smaller in a direction from the inlet (10b) to the outlet (10c); and
pushing means (30) for pushing the intraocular lens (40) in the direction from the inlet (10b) to the outlet (10c) of the tube (10);
**characterized in that** the instrument includes
a shoulder (11a) formed in the tapered inner wall portion (11) and having a height equal to or larger than a thickness of an edge of an optical part of the intraocular lens (40),
wherein the shoulder (11a) is adapted to catch one edge (41'a) of the optical part of the intraocular lens (40) so as to restrict rolling of the lens (40) in one of the rolling directions along the tapered inner wall portion (11) but allow rolling of the lens (40) in the other of the rolling directions while the lens (40) is moved by the pushing means (30), and
the shoulder (11a) is formed in a tapering direction with a length enough to make the other edge (41'b) of the optical part of the lens (40) move on an inward side of the one edge (41'a) of the lens (40) during the rolling of the lens (40) so as to roll the lens (40) into a spiral state.

2. The intraocular lens injection instrument according to claim 1, wherein the shoulder (11a) is continuously formed in the tapered inner wall portion (11) in the tapering direction over its entire length.

3. The intraocular lens injection instrument according to claim 1 or 2, wherein the shoulder (11a) has a straight stepped portion in a cross-section perpendicular to a center axis of the tube (10), and the tapered inner wall portion (11) except for the straight stepped portion of the shoulder (11a) has a curved portion in the cross-section perpendicular to the center axis of the tube (10).

## Patentansprüche

1. Einführungsinstrument einer intraokularen Linse zum Einführen einer faltbaren intraokularen Linse (40) in ein Auge, mit:
einer Einsetzröhre (10), die durch einen Einschnitt einsetzbar ist, der in einem Augapfel gemacht ist, wobei die Röhre (10) einen Einlaß (10b), einen Auslaß (10c) und einen angeschrägten inneren Wandabschnitt (11) mit einem Innendurchmesser, der allmählich in einer Richtung von Einlaß (10b) zu dem Auslaß (10c) kleiner wird, aufweist; und
einem Schiebemittel (30) zum Schieben der intraokularen Linse (40) in der Richtung von dem Einlaß (10b) zu dem Auslaß (10c) der Röhre (10);
**dadurch gekennzeichnet, daß** das Instrument enthält:
eine Schulter (11a), die in dem angeschrägten inneren Wandabschnitt (11) gebildet ist und eine Höhe gleich oder größer als eine Dicke einer Kante eines optischen Teiles der intraokularen Linse (40) aufweist,
worin die Schulter (11a) ausgelegt ist zum Ergreifen einer Kante (41'a) des optischen Teiles der intraokularen Linse (40) so, daß Rollen der Linse (40) in eine der Rollrichtungen entlang des angeschrägten inneren Wandabschnittes (11) beschränkt ist, aber das Rollen der Linse (40) in die andere der Rollrichtungen ermöglicht ist, während die Linse (40) durch das Schiebemittel (30) bewegt wird, und
die Schulter (11a) in einer Anschrägungsrichtung mit einer Länge gebildet ist, die ausreichend zum Bewirken ist, daß die andere Kante (41'b) sich auf einer Innenseite der einen Kante (41'a) der Linse (40) während des Rollens der Linse (40) bewegt, so daß die Linse (40) in einen Spiralzustand gerollt wird.

2. Einführungsinstrument einer intraokularen Linse nach Anspruch 1, bei dem die Schulter (11a) kontinuierlich in dem angeschrägten Innenwandabschnitt (11) in der Anschrägungsrichtung über seine gesamte Länge gebildet ist.

3. Einführungsinstrument einer intraokularen Linse nach Anspruch 1 oder 2, bei dem die Schulter (11a) einen geraden gestuften Abschnitt im Querschnitt senkrecht zu einer Zentralachse der Röhre (10) aufweist und der angeschrägte Innenwandabschnitt (11) mit der Ausnahme des geraden gestuften Abschnittes der Schulter (11a) einen gekrümmten Abschnitt im Querschnitt senkrecht zu der Zentralachse der Röhre (10) aufweist.

## Revendications

1. Instrument d'implantation de lentille intraoculaire destiné à implanter une lentille intraoculaire pliable (40) dans un oeil, comprenant :
un tube d'insertion (10) qui peut être inséré à travers une incision réalisée dans un globe oculaire, le tube (10) comprenant une entrée (10b), une sortie (10c), et une partie formant paroi interne conique (11) présentant un diamètre interne devenant progressivement plus petit dans une direction de l'entrée (10b) à la sortie (10c) ; et
des moyens de poussée (30) destinés à pousser la lentille intraoculaire (40) dans la direction de l'entrée (10b) à la sortie (10c) du tube (10) ;
**caractérisé en ce que** l'instrument comprend
un épaulement (11a) formé dans la partie formant paroi interne conique (11) et présentant une hauteur égale ou supérieure à l'épaisseur d'un bord d'une partie optique de la lentille intraoculaire (40),
dans lequel l'épaulement (11a) est adapté pour saisir un bord (41'a) de la partie optique de la lentille intraoculaire (40) de façon à restreindre l'enroulement de la lentille (40) dans l'une des directions d'enroulement le long de la partie formant paroi interne conique (11) mais de façon à permettre l'enroulement de la lentille (40) dans l'autre des directions d'enroulement tandis que la lentille (40) est déplacée par les moyens de poussée (30), et
l'épaulement (11a) est formé dans une direction conique avec une longueur suffisante pour permettre de déplacer l'autre bord (41'b) de la partie optique de la lentille (40) sur un côté interne du bord (41'a) de la lentille (40) pendant l'enroulement de la lentille (40) de façon à enrouler la lentille (40) dans un état de spirale.

2. Instrument d'implantation de lentille intraoculaire selon la revendication 1, dans lequel l'épaulement (11a) est formé en continu dans la partie formant paroi interne conique (11) dans la direction conique sur toute sa longueur.

3. Instrument d'implantation de lentille intraoculaire selon la revendication 1 ou 2, dans lequel l'épaulement (11a) présente une partie étagée rectiligne dans une section transversale perpendiculaire à l'axe central du tube (10), et la partie formant paroi interne conique (11), à l'exception de la partie étagée rectiligne de l'épaulement (11a), présente une partie incurvée dans la section transversale perpendiculaire à l'axe central du tube (10).
